(19) Europäisches Patentamt
European Patent Office
Office européen des brevets

(11) **EP 0 968 196 B1**

(12) **FASCICULE DE BREVET EUROPEEN**

(45) Date de publication et mention de la délivrance du brevet:
**27.10.2004 Bulletin 2004/44**

(21) Numéro de dépôt: **98941465.1**

(22) Date de dépôt: **27.07.1998**

(51) Int Cl.[7]: **C07D 249/12**, C07D 249/08, C07C 311/48, C07C 317/44, C08F 28/02, C08F 26/06, C09K 3/00, H01M 10/40, C07B 41/00, C08F 4/00

(86) Numéro de dépôt international:
**PCT/FR1998/001664**

(87) Numéro de publication internationale:
**WO 1999/005126 (04.02.1999 Gazette 1999/05)**

# (54) COMPOSES IONIQUES PERFLUOROVINYLIQUES ET LEUR UTILISATION DANS DES MATERIAUX CONDUCTEURS

IONISCHE PERFLUORVINYLVERBINDUNGEN UND IHRE VERWENDUNG IN LEITENDEN MATERIALIEN

PERFLUOROVINYL IONIC COMPOUNDS AND THEIR USE IN CONDUCTIVE MATERIALS

(84) Etats contractants désignés:
**DE FR GB IT**

(30) Priorité: **25.07.1997 CA 2212974**

(43) Date de publication de la demande:
**05.01.2000 Bulletin 2000/01**

(73) Titulaires:
- **ACEP INC.**
  **Montreal, Quebec H2Z 1A4 (CA)**
- **CENTRE NATIONAL DE LA RECHERCHE SCIENTIFIQUE (CNRS)**
  **75016 Paris (FR)**
- **RHODIA CHIMIE**
  **92408 Courbevoie Cédex (FR)**
- **L'UNIVERSITE DE MONTREAL**
  **Montréal, Québec H3C 3S7 (CA)**

(72) Inventeurs:
- **ARMAND, Michel**
  **Montréal, Québec H3T 1N2 (CA)**
- **MICHOT, Christophe**
  **F-38000 Grenoble (FR)**

(74) Mandataire: **Sueur, Yvette et al**
**Cabinet SUEUR et L'HELGOUALCH,**
**109, boulevard Haussmann**
**75008 Paris (FR)**

(56) Documents cités:
**EP-A- 0 850 920**
**EP-A- 0 850 933**
**WO-A-96/39379**
**US-A- 5 463 005**

- **DESMARTEAU D D: "Novel perfluorinated ionomers and ionenes" JOURNAL OF FLUORINE CHEMISTRY, vol. 72, no. 2, juin 1995, pages 203-8, XP002083040**
- **DATABASE WPI Section Ch, Week 9308 Derwent Publications Ltd., London, GB; Class E12, AN 93-062887 XP002083041 -& JP 05 013070 A (HITACHI MAXELL KK)**

EP 0 968 196 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet des membranes constituées par un polymère obtenu à partir de composés perfluorovinyliques ioniques, ainsi que leurs applications.

On connaît les polyélectrolytes de type polyanions incorporant des fonctions de type sulfonate ou carboxylate en tant que résines échangeuses d'ions (acide polyacrylique, acide polystyrène sulfonique éventuellement réticulé par le divinylbenzène). Ces polyélectrolytes sont dissociés uniquement en présence d'eau ou de solvants protiques très polaires comme les polyalcools, tels l'éthylène glycol ou le glycérol. Les groupements acides correspondants (carboxyliques ou sulfoniques) ne présentent pas de propriétés catalytiques marquées du fait de l'absence de gonflement de la résine et de la forte association des paires d'ions. Sous forme de membranes, ces polymères ne possèdent qu'une stabilité médiocre dans les conditions de fonctionnement d'une pile à combustible hydrogène-air ; ils sont en particulier rapidement dégradés par les espèces oxydantes présentes du côté de l'électrode à oxygène. De même, ces polymères ne peuvent être utilisés dans les procédés membranaires, tels que le procédé électrochimique chlore soude.

On connaît par ailleurs les membranes perfluorées (Nafion®) portant des groupements sulfoniques dont la stabilité chimique est bonne dans les conditions de fonctionnement d'une pile à combustible, ainsi que pour le procédé chlore-soude. Ces matériaux sont des copolymères de tétrafluoroéthylène (TFE) et d'un co-monomère portant des fonctions sulfonyles. Cependant, l'impossibilité de réticuler ces polymères impose de garder une densité de groupements ioniques faible afin d'éviter que les polymères résultants ne soient solubles ou gonflés par l'eau de manière excessive, avec pour résultat une tenue mécanique médiocre et une conductivité relativement limitée. De plus, ces membranes présentent une forte perméabilité aux gaz (oxygène et hydrogène) et à certains solvants comme le méthanol, ce qui nuit au rendement énergétique des piles à combustible, plus spécialement celles de type méthanol-air ("crossover"). Par ailleurs, bien que les groupements sulfonates attachés à des groupements perfluorés soient partiellement dissociés dans les solvants aprotiques et que les polymères solvatants soient particulièrement intéressants pour les batteries secondaires dans lesquelles les réactions aux électrodes font intervenir les ions lithium, la conductivité des gels obtenus par gonflement des membranes Nafion® par les solvants aprotiques, seuls ou en mélange, ainsi que la conductivité des mélanges de ces polyélectrolytes avec des polyéthers à base d'oxyde d'éthylène restent trop faibles. La fraction importante de segments perfluorés $-CF_2CF_2-$ issus du co-monomère TFE rend par ailleurs ces composés sensibles à la réduction à des potentiels proches de ceux de l'électrode négative, amenant une destruction du polymère. De surcroît, la chimie de ces polymères est complexe et onéreuse, le rendement de fabrication du monomère de type perfluorovinyléther :

$$CF_2=CF-O-[CF_2C(CF_3)]_p-O-CF_2CF_2SO_2F, \qquad 0 \le p \le 5$$

par cracking thermique des perfluoropolyéthers-fluorure d'acide obtenus par addition de $CF_3CF=CF_2$ sur les sultones isomérisées est faible et limite l'emploi de ces matériaux.

Les polymères qui contiennent des anions fixés à la trame du polymère et qui sont éventuellement plastifiés ou gélifiés par un solvant de type polaire sont d'un grand intérêt pour les systèmes électrochimiques tels que les batteries primaires ou secondaires, les supercapacités ou les systèmes de modulation de la lumière (vitrages électrochromes). De tels polymères sont principalement des dérivés de l'oxyde d'éthylène, de l'acrylonitrile, de polyesters de type acrylate ou méthacrylate d'alkyle ou d'oxa-alkyle, du fluorure de vinylidène. L'obtention de monomères portant des fonctions anioniques très délocalisées pouvant être incorporées, soit par co-polymérisation, soit par co-réticulation ou bien encore par mélange de polymères, dans des matériaux macromoléculaires tels que ceux utilisés dans les systèmes électrochimiques décrits ci-dessus présente donc un grand intérêt. Les monomères ioniques décrits plus haut comme composants des membranes de type Nafion® ne peuvent convenir pour cet usage, car la fraction élevée de segments perfluorés nécessaire pour obtenir le maximum de conductivité dans les milieux aprotiques ($\approx 1M.l^{-1}$) correspond à une constante diélectrique diminuée à proximité des ions et à une rigidité segmentaire accrue qui sont défavorables au mouvement des ions. De surcroît, les groupes sulfonates sont insuffisamment dissociés en comparaison avec les sels d'anions délocalisés à partir de centres azotés ou du carbone comme par exemple l'anion répondant à la formule $(R_FSO_2)X(SO_2R'_F)^-$ dans laquelle X est N, C-R ou $C-SO_2R''_F$, $R_F$, $R'_F$ et $R''_F$ sont choisis parmi le fluor et les groupements fluorés monovalents, ou bien $R_F$ et $R'_F$ forment les éléments d'un cycle divalent, et R = H ou un radical organique monovalent quelconque.

W. Navarrini, et al, (US-A-5,103,049) décrivent des méthodes de préparation de composés $R_f-CF=CF-SO_2F$ dans lesquels $R_f$ est F ou un groupement perfluoroalkyle de 1 à 9 atomes de carbones. Parmi ces composés, seul $CF_2=CF-SO_2F$ est susceptible de servir de base à des monomères pouvant polymériser sans contraintes stériques. Cependant, ce matériau s'est montré trop réactif pour servir de précurseur à des anions et à des sels monomères, du fait que l'addition nucléophile sur la double liaison C=C, appauvrie en électrons à la fois par les atomes de fluor et par

le groupement $-SO_2F$, se produit en général plus rapidement que la substitution du fluor du groupement $SO_2F$, interdisant ainsi l'accès à des monomères ioniques ou à des précurseurs de composés ioniques ("Studies of the Chemistry of Perfluorovinylsulfonyl Fluoride", Forohar Farhad, Clemson University, Thesis 1990 UMI 9115049). En particulier, les méthodes de préparation de composés anioniques utilisées avec les composés $R_FSO_2F$ sont inapplicables au composé $CF_2=CF-SO_2F$. Un procédé consistant à fixer un groupement perfluorovinyle sur un noyau phényle portant un groupement $SO_2F$ a été proposé par C. Stone et al, (WO/96/39379) ; mais dans ce cas également, il n'est pas possible de transformer la molécule $CF_2=CF-C_6H_4SO_2F$ en monomère anionique du fait de la sensibilité du groupement $CF_2=CF-$, plus réactif que $-SO_2F$ vis-à-vis des bases de type $OH^-$ ou $NH_3$.

Un autre procédé pour l'obtention de monomères du type TFE contenant un anion a été proposé par D. Desmarteau, et al. (US-A-5,463,005). Il consiste à préparer un composé comprenant un groupe perfluorovinyle et un groupe $SO_2F$, à protéger le groupe perfluorovinyle par addition de $Cl_2$ par exemple, à transformer le groupe $SO_2F$ en un groupe ionique, puis à déprotéger le groupe perfluorovinyle. Un tel procédé est néanmoins long et coûteux et les polymères obtenus à partir desdits monomères présentent les mêmes inconvénients que les polymères du type Nafion®, résultants de la faible teneur en ions $SO_3^-$ ou sulfonimide en l'absence de réticulation.

Le but de la présente invention est de fournir des membranes obtenues à partir de composés ioniques présentant une délocalisation étendue de la charge négative, une bonne activité en polymérisation ou en copolymérisation et permettant la réalisation de macromolécules possédant des fonctions ioniques dissociées et stables.

Une membrane selon la présente invention est constituée par un copolymère d'au moins deux monomères choisis parmi les composés ioniques dans lesquels la charge négative est fortement délocalisée, et elle est caractérisée en ce que :

- l'un au moins des monomères est choisi parmi les monomères monofonctionnels suivants : $\{[CF_2=CF-SO_2NCN]^-\}_m M^{m+}$, $\{[CF_2=CF-SO_2C(CN)_2]^-\}_m M^{m+}$, $\{[CF_2=CFCF_2-SO_2NCN]^-\}_m M^{m+}$, $\{[CF_2=CFCF_2-SO_2C(CN)_2]^-\}_m M^{m+}$, $\{[CF_2=CF-\Phi SO_2NCN]^-\}_m M^{m+}$, $\{[CF_2=CF-\Phi SO_2C(CN)_2]^-\}_m M^{m+}$, $\{[CF_2=CF-\Phi SO_2NSO_2CF_3]^-\}_m M^{m+}$, $\{[CF_2=CF-\Phi SO_2C(SO_2CF_3)_2]^-\}_m M^{m+}$, $\{[CF_2=CF-\Phi SO_2CH(SO_2CF_3)]^-\}_m M^{m+}$,

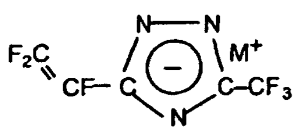
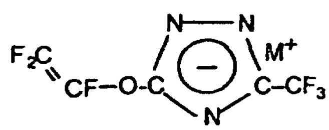
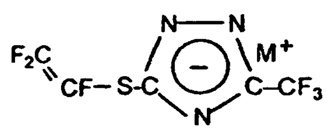
;

- l'un au moins des monomères est choisi parmi les monomères di- ou trifonctionnels suivants : $\{[(CF_2=CF-SO_2)_2N]^-\}_m M^{m+}$, $\{[(CF_2=CFCF_2-SO_2)_2N]^-\}_m M^{m+}$, $\{[(CF_2=CF-SO_2)_2CH]^-\}_m M^{m+}$, $\{[(CF_2=CFCF_2-SO_2)_2CH]^-\}_m M^{m+}$, $\{[(CF_2=CF-SO_2)_3C]^-\}_m M^{m+}$, $\{[(CF_2=CFCF_2-SO_2)_3C]^-\}_m M^{m+}$, $\{[(CF_2=CF-\Phi SO_2)_2N]^-\}_m M^{m+}$, $\{[(CF_2=CF-O-\Phi SO_2)_2N]^-\}_m M^{m+}$, $\{[(CF_2=CF-\Phi SO_2)_2CH]^-\}_m M^{m+}$, $\{[(CF_2=CF-O-\Phi SO_2)_2CH]^-\}_m M^{m+}$, $\{[(CF_2=CF-\Phi SO_2)_3C]^-\}_m M^{m+}$, $\{[(CF_2=CF-O-\Phi SO_2)_3C]^-\}_m M^{m+}$, $\{[(CF_2=CF-)_2C_6H_3SO_3]^-\}_m M^{m+}$, $\{[3,5-(CF_2=CF-)_2C_6H_3SO_2NSO_2CF_3]^-\}_m M^{m+}$

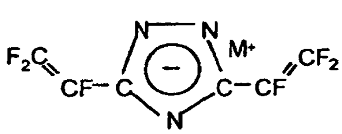
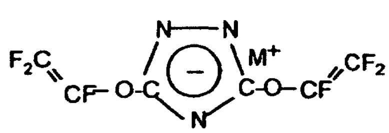

- $M^{m+}$ est le proton, ou un cation métallique ayant la valence m choisi parmi les ions des métaux alcalins, des métaux alcalino-terreux, des métaux de transition ou des terres rares, ou un cation organique onium, ou un cation organométallique, $1 \le m \le 3$ ;

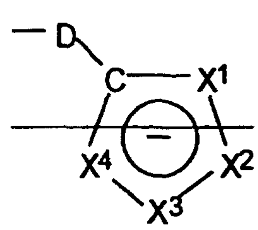

- $\Phi$ représente un groupement aromatique condensé ou non, portant ou non un ou plusieurs substituants, contenant ou non des hétéroatomes, ou un groupement polyhalogéné $-C_6H_{(4-x-y)}F_xCl_y-$ ($1 \leq x+y \leq 4$).

Le radical divalent $\Phi$ peut être un phényle $C_6H_4$ correspondant aux positions ortho, méta et para de la substitution. Il peut être un groupement aromatique, phényle substitué et / ou à noyaux condensés contenant ou non de hétéroatomes. $\Phi$ est de préférence un groupe phényle halogéné ou un groupe phényle portant 1 à 2 substituants $CF_3$, ou un noyau pyridyle.

Lorsque $M^{m+}$ est un cation métallique, il peut être un métal alcalin (en particulier $K^+$ ou $Li^+$), un métal alcalino-terreux (en particulier $Mg^{++}$, $Ca^{++}$ ou $Ba^{++}$), un métal de transition (en particulier $Cu^{++}$, $Zn^{++}$ ou $Fe^{++}$) ou une terre rare (en particulier $Re^{+++}$).

Lorsque $M^{m+}$ est un cation onium, il peut être choisi parmi les ions ammonium $[N(Y^j)_4]^+$, les ions amidinium $RC[N(Y^j)_2]_2^+$,, les ions guanidinium $C[N(Y^j)_2]_3^+$, les ions pyridinium $[C_5N(Y^j)_6]^+$, les ions imidazolium $C_3N_2(Y^j)_5^+$, les ions imidazolinium $C_3N_2(Y^j)_7^+$, les ions triazolium $C_2N_3(Y^j)_4^+$, les ions carbonium $C_5(Y^j)_5C^+$, les ions $NO^+$ (nitrosyle) ou $NO_2^+$, les ions sulfonium $[S(Y^j)_3]^+$, les ions phosphonium $[P(Y^j)_4]^+$ et les ions iodonium $[I(Y^j)_2]^+$. Dans les différents ions onium précités, les substituants $Y^j$ d'un même cation peuvent être identiques ou différents. Ils représentent l'un des substituants indiqués ci-dessus pour Y.

Lorsque $M^{m+}$ est un cation organo-métallique, il peut être choisi parmi les métallocéniums. Il peut également être choisi parmi les cations métalliques coordonnés par des atomes tels que O, S, Se N, P ou As, portés par des molécules organiques, ces cations faisant éventuellement partie d'une trame polymérique. $M^{m+}$ peut également être un cation dérivé des groupements alkyles définis pour Y ci-dessus et limités à ceux ayant de 1 à 10 atomes de carbone, par exemple un dérivé trialkylsilyle, trialkylgermanyle ou trialkylstannyle ; dans ce cas, M est relié à $[CF_2=CF-A]$ par une liaison covalente très labile et le composé se comporte comme un sel. Le cation $M^{m+}$ peut également être l'unité de répétition d'un polymère conjugué sous forme oxydée cationique.

Parmi les membranes précitées obtenues à partir d'au moins un monomère comprenant un groupe $\Phi$, on préfère tout particulièrement celles dans lesquelless $\Phi$ est $-C_6H_4-$.

Les composés ioniques monomères utilisés pour l'élaboration des membranes de la présente invention peuvent être préparés par différentes voies de synthèse.

Dans un premier mode de réalisation, l'on fait réagir un composé comprenant le groupe perfluorovinyle avec un composé ionique ayant une partie anionique $A^1$ dont la structure est analogue à celle de la partie anionique A du composé recherché et possédant de un à 3 groupes partants.

Le procédé de préparation d'un composé monomère ionique $[CF_2=CF-A^-]_m,M^{m+}$ tel que défini ci-dessus, dans lequel on fait réagir en proportions stoechiométriques un composé organo-métallique (OM1) avec un composé ionique (CI1) en présence d'un catalyseur, est caractérisé en ce que :

- le composé organo-métallique (OM1) répond à la formule $[CF_2=CF-(CF_2)_n]_e-E$ dans laquelle :
    * E représente Li, $MgL^1$, $ZnL^1$, $CdL^1$, Cu, Mg, Zn, Cd, Hg, un groupement trialkyl-silyle, trialkyle-germanyle ou trialkyle-stannyle ;
    * n est 0 ou 1 ;
    * e représente la valence de E ;
    * $L^1$ représente un groupe partant choisi parmi les halogènes, les pseudohalogènes, y compris les sulfonates, les radicaux contenant les cycles de l'imidazole, du 1,2,4-triazole et de leurs homologues dans lesquels un ou plusieurs atomes de carbone portent des substituants, y compris ceux dans lesquels les substituants forment un cycle (par exemple un benzotriazole), les radicaux perfluoroalkylsufonyloxy et les radicaux arylsulfonyloxy ;

- le composé ionique (CI1) répond à la formule $[(LA^1)^-]_{m'}\ M'^{m'+}$ dans laquelle :
    * $M'^{m'+}$ représente un proton, ou un cation métallique ayant la valence m choisi parmi les ions des métaux alcalins, des métaux alcalino-terreux, des métaux de transition, des terres rares, ou un cation organique onium, ou un cation organo-métallique, $1 \leq m \leq 3$. M' est choisi de manière à ne pas interférer avec la réaction de formation ;
    * L a la même signification que $L^1$, étant entendu que les groupes L et $L^1$ des réactifs respectifs utilisés lors d'une réaction peuvent être identiques ou différents ;
    * $A^1$ représente un groupement anionique répondant à l'une des formules suivantes :

$$[-(CF_2)_n-SO_2Z^1]^-$$

$[-(O)_{n'}-\Phi SO_2Z^1]^-$

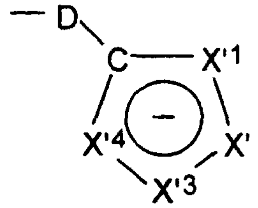

dans lesquelles :

- n et n' représentent 0 ou 1 ;
- D représente une simple liaison, un atome d'oxygène, un atome de soufre, un groupe carbonyle -CO- ou un groupe sulfonyle $-SO_2-$ ;
- $Z^1$ représente l'oxygène -O (sauf si n ou n' sont nuls) ou l'un des groupements $=NC\equiv N$, $=C(C\equiv N)_2$, $=NSO_2R^1$, $=C[SO_2R^1]_2$ ;
- les groupements $X'^1$ à $X'^4$, désignés ci-après par $X'^i$, représentent N, $C-C\equiv N$, $CR^1$, $CCOR^1$ ou $CSO_2R^1$, étant entendu que, dans un groupement pentacyclique, les $X'^i$ peuvent être identiques ou différents ;
- $R^1$ représente Y, YO-, YS-, $Y_2N-$, F, $CF_2=CF-$, $CF_2=CFCF_2-CF_2=CF-O-$ ou $R_F = C_qF_{2q+1}$ ($0 \leq q \leq 12$), ou un groupe partant choisi parmi les halogènes, les pseudohalogènes, y compris les sulfonates, les radicaux contenant les cycles de l'imidazole, du 1,2,4-triazole et de leurs homologues, les radicaux perfluoroalkylsufonyloxy et les radicaux arylsulfonyloxy, étant entendu que si deux substituants $R^1$ sont présents sur un même groupement, ils peuvent être identiques ou différents, et qu'au plus deux substituants $R^1$ sont des groupes partants ;
- Y a la signification donnée ci-dessus.

Lorsque le cation M' du réactif utilisé est différent du cation M du composé final souhaité, on modifie le composé obtenu par des techniques classiques d'échange de cations.

Les groupes partants préférés L et $L^1$ sont F, Cl, Br, I, le radical imidazole, le radical trifluorométhanesulfonyloxy $CF_3SO_3$- et les radicaux arylsulfonyloxy.

Pour les composés ioniques (CI1), on préfère tout particulièrement les groupes anioniques suivants : $[(FSO_2)NSO_2R_F]^-$, $[(ClSO_2)NSO_2R_F]^-$, $[(ImSO_2)NSO_2R_F]^-$, $[(FSO_2)_2N]^-$, $[(ClSO_2)_2N]^-$, $[(ImSO_2)_2N]^-$, $[(FSO_2)NCN]^-$, $[(ClSO_2)NCN]^-$, $[(ImSO_2)NCN]^-$, $[(FSO_2)C(R)SO_2R_F]^-$ $[(ClSO_2)C(R)SO_2R_F]^-$, $[(ImSO_2)C(R)SO_2R_F]^-$, $[(FSO_2)_2CR]^-$, $[(ClSO_2)_2CR]^-$, $[(ImSO_2)_2CR]^-$, $[(FSO_2)C(CN)_2]^-$, $(ClSO_2)C(CN)_2]^-$, $(ImSO_2)C(CN)_2]^-$, $[(FSO_2)_3C]^-$, $[(ClSO_2)_3C]^-$, $[(ImSO_2)_3C]^-$, $[(Cl\Phi SO_2)NSO_2R_F]^-$, $[(Br\Phi SO_2)NSO_2R_F]^-$, $[(I\Phi SO_2)NSO_2R_F]^-$, $[(CF_3SO_3\Phi SO_2)NSO_2R_F]^-$, $[(CH_3\Phi SO_3\Phi SO_2)NSO_2R_F]^-$, $[(3,5-Br_2C_6H_3SO_3]^-$, $[(3,5-Br_2C_6H_3SO_2N(SO_2R_F)]^-$, $[(3,5-I_2C_6H_3SO_3]^-$, $[(3,5-I_2C_6H_3SO_2N(SO_2R_F)]^-$, $[(Br\Phi SO_2)NCN]^-$, $[(I\Phi SO_2)NCN]^-$, $[(CF_3SO_3\Phi SO_2)NCN]^-$, $[(CH_3\Phi SO_3\Phi SO_2)NCN]^-$ $[(Cl\Phi SO_2)C(CN)_2]^-$, $[(Br\Phi SO_2)C(CN)_2]^-$, $[(I\Phi SO_2)C(CN)_2]^-$, $[(CF_3SO_3\Phi SO_2)C(CN)_2]^-$, $[(CH_3\Phi SO_3\Phi SO_2)NC(CN)_2]^-$, $[(Cl\Phi SO_2)N(SO_2\Phi Cl)]^-$, $[(Br\Phi SO_2)N(SO_2\Phi Br)]^-$, $[(I\Phi SO_2)N(SO_2C_4H_5I)]^-$, $[(CF_3SO_3\Phi SO_2)N(SO_2\Phi SO_3CF_3)]^-$, $[(CH_3\Phi SO_3\Phi SO_2)N(SO_2\Phi SO_3\Phi CH_3)]^-$, $[(Cl\Phi SO_2)_2C(SO_2R_F)]^-$, $[(Br\Phi SO_2)_2C(SO_2R_F)]^-$, $[(I\Phi SO_2)_2C(SO_2R_F)]^-$, $[(CF_3SO_3\Phi SO_2)_2C(SO_2R_F)]^-$, $[(Cl\Phi SO_2)C(SO_2R_F)_2]^-$, $[(Br\Phi SO_2)C(SO_2R_F)_2]^-$, $[(I\Phi SO_2)C(SO_2R_F)_2]^-$, $[(CF_3SO_3\Phi SO_2)C(SO_2R_F)_2]^-$, $(CH_3\Phi SO_3\Phi SO_2)C(SO_2R_F)_2]^-$, $[(Br\Phi C(SO_2R_F)_2]^-$, $[(3,5-Br_2C_6H_3C(SO_2R_F)_2]^-$,

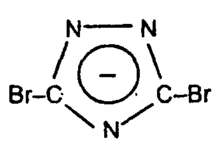 , 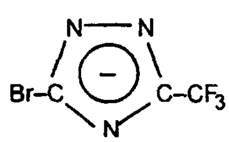 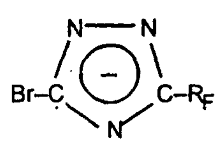

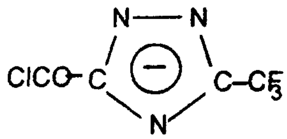 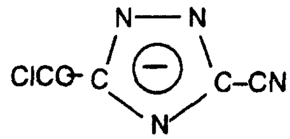 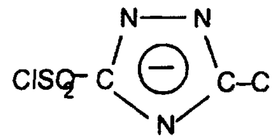

Dans les groupes anioniques précités des composés (CI1), R et $R_F$ ont la signification donnée précédemment et comprennent de préférence de 0 à 10 atomes de carbone.

Un composé (OM1) tel que $CF_2$=CF-Li est préparé par action d'une base forte B et d'un agent de lithiation R'-Li sur le tétrafluoro 1,3,3,3-éthane à basse température selon le schéma réactionnel :

$$CF_3\text{-}CF_2F + B \Rightarrow CF_2\text{=}CHF + [BHF]$$

$$CF_2\text{=}CHF + R'Li \Rightarrow CF_2\text{=}CFLi + LiF + R'H$$

Les composés $CF_2$=CF-E dans lesquels E est différent de Li peuvent être obtenus par échange d'ions.

Il est avantageux d'utiliser le butyllithium à la fois comme base forte et comme agent de lithiation.

Le composé $CF_2$=$CFCF_2$-E peut être préparé à partir du fluorosulfate de perfluoroallyle par simple substitution nucléophile selon le schéma réactionnel suivant :

$$CF_2\text{=}CFCF_2\text{-}SO_3F + M^+E^- \Rightarrow CF_2\text{=}CFCF_2\text{-}E + M^+SO_3F^-$$

Un composé $M^+E^-$ particulièrement approprié est KI.

Les composés organométalliques (OM1) sont choisis plus particulièrement parmi : $CF_2$=CFLi, $(CF_2\text{=}CF)_{2-x}Mg(Hal)_x$, $(CF_2\text{=}CF)_{2-x}Zn(Hal)_x$ $(CF_2\text{=}CF)_{2-x}Cd(Hal)_x$ ($0 \leq x < 2$) ; Hal = Cl, Br, I, pseudo-halogène), $(CF_2\text{=}CF)_{3-y}Al(Hal)_y$, ($0 \leq y < 3$) $CF_2$=CFCu, $CF_2\text{=}CFSi(CH_3)_3$, $CF_2\text{=}CFSi(C_2H_5)_3$, $CF_2\text{=}CFSn(CH_3)_3$, $CF_2\text{=}CFSn(C_2H_5)_3$, $CF_2\text{=}CFSn(C_4H_9)_3$, $CF_2\text{=}CFCF_2CdBr$, $CF_2\text{=}CFCF_2Cu$, $CF_2\text{=}CFCF_2Zn(O_3SF)$, $CF_2\text{=}CFCF_2Si(CH_3)_3$, $CF_2\text{=}CFCF_2Si(C_2H_5)_3$.

Il est connu de l'homme de l'art que la stabilité et les conditions de mise en oeuvre de composés organométalliques halogénés varient selon la nature de E ; en particulier les composés organolithiens ne sont stables qu'à des températures inférieure à -60°C et sont habituellement préparés et utilisés à la température de la neige carbonique, alors que les composés plus covalents comme les dérivés des silanes sont stables même au dessus de la température ambiante.

Le procédé est mis en oeuvre en présence d'un catalyseur choisi parmi les dérivés du nickel ou du palladium coordonné par des bases de type amine ou phosphine. A titre d'exemple, on peut citer le nickel bis-(2,2'bipyridyl), le nickel tétrakis(triphényl phosphine) et ses dérivés sulfonés, l'acétate de palladium, le tris-benzylidène-cétone-di-palladium, le palladium tétrakis (triphényl phosphine) et ses dérivés sulfonés, ainsi que les composés obtenus par remplacement de deux molécules de triphényl phosphine par $[(C_6H_5)_2PCH_2]_2$ ou $[(C_6H_5)_2PCH_2]_2CH_2$. Le nickel et le palladium ont des propriétés catalytiques pour les réactions de couplage dites de Susuki lorsque leur degré d'oxydation est supérieur ou égal à 0 et inférieur ou égal à 2.

Dans autre mode de réalisation de l'invention, on prépare un composé monomère ionique $[CF_2\text{=}CF\text{-}A^-]_m$, $M^{m+}$ selon l'invention en faisant réagir un composé comprenant un groupe fluorovinylique protégé $CF_2L^3CFL^4\text{-}(CF_2)_nE^1$ avec un réactif $[(L^5)_aA^2]_{m'}M'^{m'+}$ permettant la formation du groupement anionique A, puis l'on élimine les groupements protecteurs par une réduction chimique ou électrochimique, ou par une déhydrohalogénation. M' a la signification indiquée précédemment. $L^3$ et $L^4$ représentent H ou un halogène, un seul parmi eux étant éventuellement H. $E^1$ a la signification donnée ci-dessus pour E. $L^5$ représente un groupe partant ayant la même définition que le groupe partant L. "a" est la valence du groupe anionique $A^2$. $A^2$ représente un groupement répondant à l'une des formules suivantes $[-(CF_2)_n\text{-}SO_2Z^2]^-$, $[-(O)_n$, $-\Phi SO_2Z^2]^-$ ou

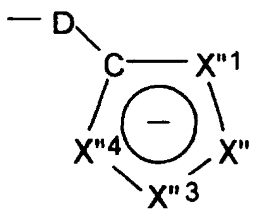

dans lesquelles :

- n, n', D et Φ ont la signification indiquée précédemment ;
- $Z^2$ représente l'oxygène -O (sauf si n ou n' sont nuls) ou l'un des groupements =NC≡N, $=C(C\equiv N)_2$, $=NSO_2R^2$, $=C[SO_2R^2]_2$ ;
- les groupements $X''^1$ à $X''^4$, désignés ci-après par $X''^i$, représentent N, C-C≡N, $CR^2$, $CCOR^2$ ou $CSO_2R^2$, étant entendu que, dans un groupement pentacyclique, les $X''^i$ peuvent être identiques ou différents ;
- $R^2$ représente OH-, SH-, Y, YO-, YS-, $Y_2N$-, F, $CF_2$=CF-, $CF_2=CFCF_2-CF_2=CF-O-$ ou $R_F = C_qF_{2q+1}$ ($0 \le q \le 12$), étant entendu que si deux substituants $R^2$ sont présents sur un même groupement, ils peuvent être identiques ou différents, et qu'au plus deux substituants $R^2$ représentent OH- ou SH- ;
- Y a la signification donnée précédemment.

A titre d'exemple, on peut citer la réaction suivante : $(ClSO_2)_2NK + 2\ CF_2Cl\text{-}CFClLi \Rightarrow (CF_2Cl\text{-}CFClSO_2)_2NK + 2LiCl$.

Lorsque le cation M' du réactif utilisé est différent du cation M du composé final souhaité, on modifie le composé obtenu par des techniques classiques d'échange de cations. La plus grande résistance des composés ainsi protégés vis-à-vis des nucléophiles, en particulier les bases, permet d'effectuer des réactions sur des molécules servant d'intermédiaire à la préparation des anions monomères, réactions qui seraient impossibles si la double liaison éthylénique n'était pas protégée. Il est en particulier possible de former des intermédiaires de type sulfamide ou hydrazide permettant la construction des espèces anioniques.

Par exemple, l'anion dérivé par addition du chlore sur l'acide trifluoroacrylique peut être transformé en groupement triazole par l'intermédiaire d'un hydrazide. L'anion triazole, plus dissocié que l'anion carboxyle, est obtenu selon le schéma simplifié:

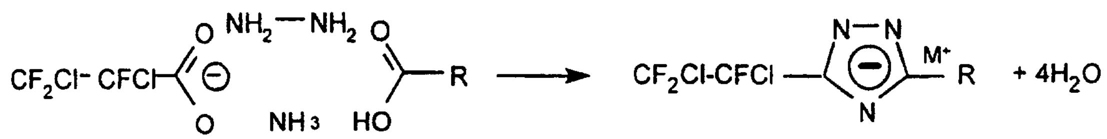

L'acide acrylique fluoré de départ se prépare aisément par action de $CO_2$ sur un dérivé organométallique tel $CF_2$=CFLi, lui-même obtenu aisément par action d'un alkyllithium sur le composé commercial trifluoro-1,1,1,2-tétrafluoroéthane commercialisé sous la marque KLEA®.

Des réactions similaires sont possibles avec un composé contenant un groupe anionique à base de soufre, tel qu'un sulfonate. Elles permettent d'obtenir, par l'intermédiaire d'un sulfamide, un composé comprenant un groupement anionique sulfonylimide plus dissocié que le groupement sulfonate, par les étapes réactionnelles suivantes :

$$\text{sulfonate + agent chlorant} \Rightarrow RSO_2Cl$$

$$RSO_2Cl + NH_3 \Rightarrow RSO_2NH_2\ ;$$

$$RSO_2NH_2 + R'SO_2Cl \Rightarrow RSO_2N(H)SO_2R'$$

Ces réactions ne peuvent être mises en oeuvre si R = $CF_2$=CF. En revanche, on obtient le résultat souhaité si R = $CF_2$Cl-CFCl et par exemple R' = R ou $CF_3$. Le sulfonate est obtenu facilement par les réactions suivantes :

$$CF_2Cl\text{-}CFClLi + ClSO_3Si(CH_3)_3 \Rightarrow CF_2Cl\text{-}CFSO_3Li + ClSi(CH_3)_3.$$

Un composé $[CF_2L^2\text{-}CFL^3\text{-}(CF_2)_n]\text{-}E^1$, en particulier $CF_2ClCFClLi$, peut être obtenu par réaction du composé $[CF_2=CF\text{-}(CF_2)_n]\text{-}E^1$ correspondant avec un composé $L^2L^3$ à une température comprise entre -80°C et 110°C, par exemple dans le mélange de Trapp (composition THF/éther/pentane). Parmi les composés $L^2L^3$ qui s'additionnent facilement sur la double liaison perfluorovinyle, l'on peut citer FCl, $Cl_2$, ClBr, $Br_2$, ICl, IBr, $I_2$, $(SCN)_2$, HCl, HBr et HI. Les composés (OM2) lithiens peuvent servir de base à la préparation d'autres composés organométalliques, tels que les dérivés du silicium ou du zinc, par échange.

De préférence les couples ($L^2$) / ($L^3$) et $L^2H$ sont choisis parmi $Cl_2$, $Br_2$, FCl, FBr BrCl, ICl,HF, HCl et HBr, $Cl_2$ et HCl étant particulièrement préférés.

Les groupements $L^2L^3$ sont facilement éliminés par réduction ou par déhydrohalogénation. Le réducteur est avantageusement choisi parmi le zinc, le couple cuivre-zinc, les sels de $Ti^{3+}$, $V^{2+}$, $Cr^{+2}$, $Sm^{2+}$, et le tétrakis (diméthylaminoéthylène) $\{[(CH_3)_2N]_2C=\}_2$. Une réduction électrochimique peut également être mise en oeuvre, directement ou par l'intermédiaire des métaux précédents servant de médiateurs. Les agents de déhydrohalogénation sont choisis parmi les bases fortes et sont connus de l'homme de l'art. On peut citer en particulier NaH [utilisé éventuellement en présence de bases phosphorées de type phosphazène P1-P4 (Fluka AG, Bâle, catalogue N° 79408, 79412, 79417, 79421, 422 79432)], $(CH_3)_3CONa$, $(CH_3)_3COK$, le LDA (lithium-di-isopropylamide), $[(CH_3)_2CH]_2NLi$, les dérivés des hexaalkyl-disilazanes, en particulier $[(CH_3)_3Si]_2NLi$, $[(CH_3)_3Si]_2Nna$ et $[(CH_3)_3Si]_2NK$.

Dans un autre mode de réalisation, l'on fait réagir un composé ionique ayant une partie anionique $A^3$ dont la structure est analogue à celle de la partie anionique A du composé recherché et possédant de un à 3 groupes hydroxyle ou thiol, sur un composé comprenant le groupe perfluorovinyle.

L'invention a pour objet un procédé de préparation d'un composé monomère ionique $[CF_2=CF\text{-}A^-]_{m'}M^{m+}$ tel que défini ci-dessus, caractérisé en ce que l'on fait réagir en proportions stoechiométriques un composé $CF_2=CFL^2$ dans lequel $L^2$ représente Cl, Br ou F, avec un composé ionique (CI2) $[(HQA^3)^-]_{m'}$ $(M')^{m'+}$, et en ce que l'on traite le produit d'addition obtenu par une base forte B.

Le schéma réactionnel est le suivant:

$$CF_2=CFL^2 + [(HQA^3)^-] \Rightarrow + HCF_2\text{-}CFL^2\text{-}Q\text{-}A^3$$

$$HCF_2\text{-}CFL^2\text{-}Q\text{-}A^3 + Base \Rightarrow + CF_2=CF\text{-}Q\text{-}A^3 + BaseHL$$

La partie cationique $(M')^{m'+}$ a la signification donnée précédemment, les cations de métal alcalin étant préférés.

La partie anionique $(HQA^3)^-$ est telle que :

- Q représente O ou S ;
- $A^3$ représente un groupement anionique répondant à l'une des formules suivantes :

$$[\text{-}(CF_2)_n\text{-}SO_2Z^3]^-$$

$$[\text{-}\Phi SO_2Z^3]^-$$

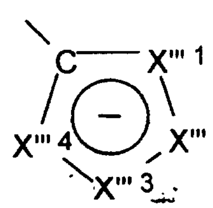

dans lesquelles :

- n et Φ ont la signification indiquée précédemment ;
- $Z^3$ représente l'oxygène =O (sauf si n est nul) ou l'un des groupements =NC≡N, $=C(C≡N)_2$, $=NSO_2R^3$, $=C[SO_2R^3]_2$ ;
- les groupements X'''1 à X'''4, désignés ci-après par X'''i, représentent N, C-C≡N, $CR^3$, $CCOR^3$ ou $CSO_2R^3$, étant entendu que, dans un groupement pentacyclique, les X'''i peuvent être identiques ou différents ;
- $R^3$ représente HO-, HS-, Y, YO-, YS-, $Y_2N$-, F, $CF_2=CF$-, $CF_2=CFCF_2$-, $CF_2=CF-O$- ou $R_F = C_qF_{2q+1}$ ($0 \leq q \leq 12$), étant entendu que si deux substituants $R^3$ sont présents sur un même groupement, ils peuvent être identiques ou différents, et qu'au plus deux substituants $R^3$ sont des groupes -OH ou -SH ;
- Y est tel que défini précédemment.

La base forte B peut être en particulier NaH [utilisé éventuellement en présence de bases phosphorées de type phosphazène P1-P4 (Fluka AG, Bâle, catalogue N° 79408, 79412, 79417, 79421, 422 79432)], $(CH_3)_3CONa$, $(CH_3)_3COK$, le LDA (lithium-di-isopropylamide), $[(CH_3)_2CH]_2NLi$, les dérivés des hexaalkyl-disilazanes, en particulier $[(CH_3)_3Si]_2NLi$, $[(CH_3)_3Si]_2Nna$ et $[(CH_3)_3Si]_2NK$. Le t-butoxyde de potassium est particulièrement approprié.

Lorsque le composé ionique (CI2) comprend un thiol, il est possible de transformer par oxydation le sulfure de l'anion $[HCF_2-CFL^2SA]^-$ du produit intermédiaire obtenu en sulfone. Le pouvoir électroattracteur de la sulfone contribue à diminuer la basicité de l'anion correspondant $[HCF_2-CFL^2-SO_2.A]^-$ et l'on peut alors éliminer $L^2H$ dans des conditions douces, en présence d'une base azotée tertiaire par exemple.

Les composés (CI2) préférés sont ceux qui contiennent l'un des anions suivants : $[\{HO-(\Phi)SO_2\}N(SO_2R_F)]^-$, $[\{HS-(\Phi)SO_2\}N(SO_2R_F)]^-$, $[\{HO-(\Phi)SO_2\}NCN]^-$, $[\{HO(\Phi)SO_2\}NCN]^-$, $[\{HS(\Phi)SO_2\}NCN]^-$, $[\{OH(\Phi)SO_2\}C(CN)_2]^-$, $[\{HS(\Phi)SO_2\}C(CN)_2]^-$, $[\{HO(\Phi)SO_2\}N\{SO_2(\Phi)OH\}]^-$, $[\{HS(\Phi)SO_2\}N(\Phi)SH]^-$, $[\{HO(\Phi)SO_2\}_2C(R)]^-$, $[\{HO(\Phi)SO_2\}_2C(SO_2R)]^-$, $[\{HO(\Phi)SO_2\}_2C(SO_2R_F)]^-$, $[\{HO(\Phi)SO_2\}C(SO_2R_F)_2]^-$, $[\{HS(\Phi)SO_2\}_2C(R)]^-$, $[\{HS(\Phi)SO_2\}_2C(SO_2R)]^-$, $[\{HS(\Phi)SO_2\}_2C(SO_2R_F)]^-$, $[\{HS(\Phi)SO_2\}C(SO_2R_F)_2]^-$, $[(3,5-(HO)_2-C_6H_3SO_3]^-$ $[(3,5-(HO)_2-C_6H_3SO_2)N(SO_2R_F)]^-$, $[(3,5-(HO)_2-C_6H_3C(SO_2R_F)]^-$, $[\{HO-(\Phi)SO_2\}_2C(SO_2R_F)]^-$, $[\{HS-(\Phi)SO_2\}_2C(SO_2R_F)]^-$,

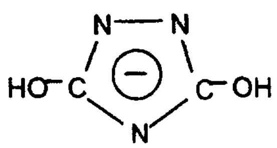

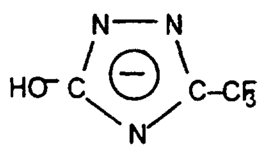

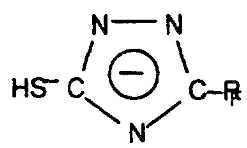

Dans un autre mode de réalisation, les composés $[CF_2=CF-A^-]_{m'}M^{m+}$ sont préparés par un procédé, caractérisé en ce qu'il consiste à faire réagir en proportions stoechiométriques un composé $L^6CF_2CF_2L^6$ avec un composé ionique $[(HQA^3)^-]_{m'}(M')^{m'+}$, et à réduire le composé de substitution obtenu soit chimiquement, soit électrochimiquement. Le composé $[(HQA^3)^-]_{m'}(M')^{m'+}$ est tel que défini précédemment. $L^6$ représente un groupe partant choisi parmi les halogènes, les pseudohalogènes et les sulfonates. Les halogènes sont particulièrement préférés

Les composés de substitution obtenus sont soumis à une réduction chimique ou une réduction électrochimique pour éliminer les groupes partants $L^6$. La réduction peut être catalysée par le zinc, le couple cuivre-zinc, les sels de $Ti^{3+}$, $V^{2+}$, $Cr^{+2}$, $Sm^{2+}$, et le tétrakis (diméthylaminoéthylène) $\{[(CH_3)_2N]_2C=\}_2$. Une réduction électrochimique peut être mise en oeuvre, directement ou par l'intermédiaire des métaux précédents servant de médiateurs.

Les composés ioniques de la présente invention, qui contiennent tous au moins un groupement $CF_2=CF$-, peuvent être polymérisés par voie radicalaire. La polymérisation peut être effectuée en solution ou en émulsion avec des amorceurs radicalaires classiques tels que les peroxydes, les composés azoïques, les persulfates, les photoamorceurs de type benzoïne ou autres. Lors de l'élaboration de matériaux réticulés, la masse moléculaire entre noeuds de réseaux n'est pas nécessairement très élevée, ce qui constitue un avantage important par rapport aux matériaux du type Nafion.

Un polymère selon la présente invention est constitué par une partie polyanionique à laquelle sont associés des cations en nombre suffisant pour assurer la neutralité électronique du polymère, la partie polyanionique étant constituée par des unités récurrentes :

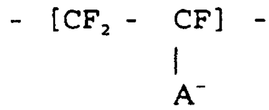

dans lesquelles A a la signification donnée ci-dessus dans la définition des composés monomères de la présente invention.

Un polymère de l'invention peut également être constitué par des unités récurrentes

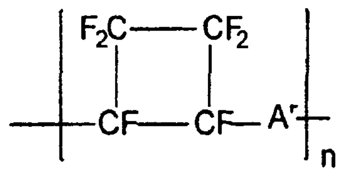

Un tel polymère est obtenu par polymérisation d'un composé difonctionnel selon l'invention dans lequel le groupement anionique A- contient lui-même un radical perfluorovinyle.

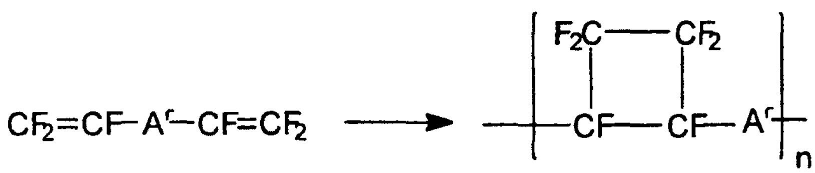

Un tel composé $[(CF_2{=}CF)_2\text{-}A'^{-}]_m,M^{m+}$ dans lequel M a la signification donnée précédemment et A' représente un anion répondant à l'une des formules (I), (II) ou (III) mentionnées précédemment dans lesquelles un substituant Z ou $X^i$ contient un radical perfluorovinyle. Dans ce cas, deux radicaux perfluorovinyles forment un cycle à 4 carbones relié à un cycle analogue par la partie anionique A' pour former un polymère linéaire.

La polymérisation peut être effectuée à partir des seuls composés de l'invention, et l'on obtient un homopolymère dans lequel chacune des unités récurrentes porte un groupement ionique.

Lorsque la polymérisation est effectuée en présence d'un comonomère, il est possible de limiter la quantité de groupements ioniques sur le copolymère.

La polymérisation de monomères monofonctionnels permet d'obtenir des polymères linéaires. La polymérisation de monomères di- ou tri-fonctionnels, dans lesquels le groupe anionique A contient lui-même un ou deux groupes $CF_2{=}CF$-supplémentaires, permet d'obtenir des matériaux macromoléculaires réticulés. Dans ce cas également, une copolymérisation avec un comonomère ne portant pas de groupements ioniques permet d'ajuster le nombre de groupes fonctionnels introduits.

De manière générale, lorsque les composés de la présente invention sont polymérisés en présence d'un comonomère, le choix du monomère, du comonomère, du nombre de groupes polymérisables sur le monomère et le comonomère permet d'ajuster les propriétés des matériaux macromoléculaires obtenus en fonction de l'usage qui en est prévu.

Un copolymère obtenu par copolymérisation d'un mélange de composés monofonctionnels et de composés difonctionnels selon la présente invention est utile pour l'élaboration de membranes. L'homopolymérisation ou la copolymérisation des monomères selon l'invention possédant une fonction perfluorovinylique conduit à des polymères linéaires. Par addition de monomères possédant plus d'une fonction polymérisable, il est facile d'obtenir des réseaux réticulés. Ces matériaux peuvent être utilisés pour élaborer des membranes ayant une tenue mécanique améliorée, dans lesquelles on peut contrôler les taux de gonflement et la perméation dans les liquides dans lesquels elles seront mises en oeuvre. L'amélioration de la tenue mécanique est aussi un facteur important pour la préparation de membranes très fines, dans lesquelles la résistance est diminuée, et pour la réduction du coût des matières premières. Le contrôle du taux de réticulation permet de même d'augmenter la concentration en ions fixés dans la membrane sans induire de solubilité ou de gonflement excessifs. Dans les procédés mettant en oeuvre des membranes, il est particulièrement avantageux de préparer la membrane dans sa forme définitive, sous forme de feuille ou de tube à partir d'une solution concentrée de monomères dans un solvant permettant des techniques d'épandage ou d'extrusion, par

copolymérisation des monomères, y compris ceux permettant la réticulation.

Pour l'élaboration de membranes, il est particulièrement avantageux d'utiliser des composés monomères de la présente invention qui présentent une grande solubilité dans les solvants dans lesquels la polymérisation sera effectuée, et une réactivité comparable à celle des doubles liaisons portées par le monomère monofonctionnel et de celles portées par les monomères polyfonctionnels permettant la réticulation, de manière à obtenir une distribution régulière des noeuds de réticulation.

Les membranes obtenues à partir des composés de la présente invention peuvent être utilisées notamment dans les systèmes de dialyse, comme séparateur dans un réacteur biphasique ou pour les procédés membranaires de type chlore-soude, ou pour la récupération d'effluents, ou comme électrolyte dans une pile à combustible. S'agissant de piles à combustible, un matériau macromoléculaire obtenu à partir de composés monomères de l'invention peut également être utilisé comme liant dans le matériau d'électrode.

La présente invention est décrite plus en détail par les exemples suivants, l'invention n'étant pas limitée à ces exemples.

**Exemple 1**

On a effectué une distillation azéotropique dans 200 ml de toluène, de 20 mmoles d'acide carboxylique $ClCF_2CFClCOOH$ et de 10 mmoles d'hydrazine monohydrate. Après 24 heures, le toluène a été évaporé et on a obtenu le composé $ClCF_2CFClCONHNHCOCFClCF_2Cl$. On a mis ensuite ce composé en solution dans 200 ml de $PCl_5$ contenant 40 mmoles d'hydrochlorure de diméthylaniline. Après avoir porté ce mélange au reflux durant 24 heures, on a obtenu après refroidissement deux phases, dont une plus dense de $ClCF_2$-CFCl-CCl=N-N=CCl-CFCl-$CF_2Cl$. Ce produit a été récupéré à l'aide d'une ampoule à décanter, lavé à l'eau, puis traité par de l'ammoniaque dans un mélange de 100 ml d'éther et de 100 ml d'une solution 4 M d'ammoniaque. Après 24 heures sous agitation, les solvants ont été évaporés, on a ainsi obtenu $ClCF_2$-CFCl--C($NH_2$)=N-N=C($NH_2$)$CFClCF_2Cl$ . Ce dernier produit a ensuite été porté au reflux dans 100 ml de butanol pendant 48 heures, puis repris après évaporation du butanol dans 100 ml de THF anhydre contenant du zinc. Après 24 heures, le solvant a été évaporé et le produit résiduel recristallisé dans une solution saturée de KCl. Après filtration, le composé recristallisé a été cobroyé avec du sulfate d'ammonium puis sublimé sous vide, on a ainsi récupéré le composé suivant :

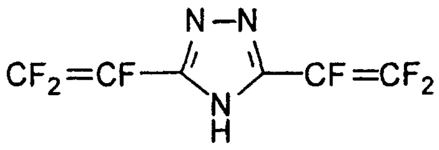

**Exemple 2**

10 mmoles d'hydrazine monohydrate ont été traitées dans le THF par 15 mmoles de $CF_3CO_2C_2H_5$. Après 8 heures, le THF a été évaporé et le produit séché. On a obtenu quantitativement $CF_3CONHNH_2$. On a alors effectué une distillation azéotropique dans 200 ml de toluène de ce composé avec 10 mmoles de l'acide carboxylique $ClCF_2CFClCOOH$. Après 24 heures, on a évaporé le toluène et on a obtenu $ClCF_2CFClCONHNHCOCF_3$. Ensuite, on a traité ce composé par un procédé analogue à celui décrit dans l'exemple 1 pour le composé $ClCF_2CFClCONHNHCOCFClCF_2Cl$, et on a obtenu le composé suivant :

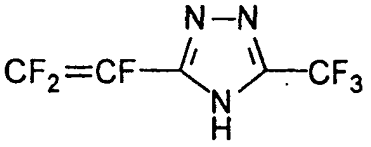

**Exemple 3**

Dans 30 ml de THF anhydre à 0°C et sous argon contenant 20 mmoles de zinc, on a ajouté lentement 10 mmoles de l'iodure de trifluorovinyle $CF_2$=CFI. Après deux heures sous agitation, l'excès de zinc a été éliminé par filtration sous argon. On a alors ajouté à la solution de zincique 5 mmoles du sel de lithium du 2,5-dibromo-1,3,4-triazole et 1 mmoles de $Pd[P(C_6H_5)_3]_4$ comme catalyseur. Après 24 heures sous agitation, le solvant a été évaporé puis le résidu cobroyé avec de l'hydrogéno sulfate d'ammonium. Après sublimation de ce mélange, on a obtenu le composé suivant :

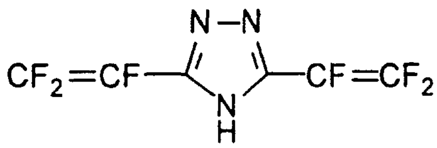

**Exemple 4**

Dans un réacteur chimique, on a introduit 10 mmoles du sel de lithium de l'urazole et 500 µmoles de bis-diméthylamino-1,8-anthracène dans 50 ml de THF. Après avoir porté le milieu réactionnel à -20°C, on a introduit lentement dans le réacteur 20 mmoles de tétrafluoroéthylène (PCR). Après 24 heures, on a effectué un balayage d'argon dans le milieu réactionnel puis on a laissé le milieu remonter lentement à température ambiante. On a alors ajouté lentement 30 mmoles de tert butoxyde de sodium en solution dans 20 ml de THF anhydre. Après 3 heures, le solvant a été évaporé, le résidu a été recristallisé dans une solution saturé de KCl puis sublimé après avoir été cobroyé avec du sulfate d'ammonium. On a obtenu le composé suivant :

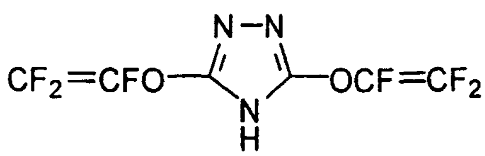

**Exemple 5**

Par un procédé similaire à celui décrit dans l'exemple 4, on a fait réagir 10 mmoles de 1-trifluorométhyl-3-hydroxy-2,4,5-triazole avec 10 mmoles de tétrafluoroéthylène. On a ainsi obtenu le composé suivant :

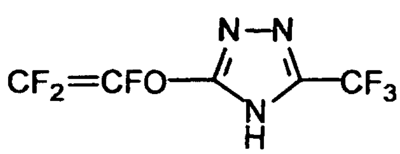

**Exemple 6**

On a condensé 24,5 g de 1,1,1,2-tétrafluoroéthane dans 300 ml d'éther anhydre à -78°C. On a ensuite ajouté goutte à goutte sous agitation 44 ml d'une solution 10M de butyllithium dans l'hexane. Après une heure, on a ajouté au mélange réactionnel 250 ml d'une solution commerciale 1M de chlorure de zinc dans l'éther. La réaction s'est effectuée selon le schéma réactionnel suivant :

$$CF_3CH_2F + C_4H_9Li \Rightarrow C_4H_{10} + CF_2{=}CFLi + HF$$

$$CF_2{=}CFLi + ZnCl_2 \Rightarrow CF_2{=}CFZnCl + LiCl$$

La suspension contenant le trifluorovinyle zincique a été ramenée à température ordinaire et 45 g du sel de sodium de bis(bromophénylsulfonimide) (préparé selon le mode opératoire de l'exemple 7) dans 150 ml de diméthylformamide anhydre ont été ajoutés, ainsi que 800 mg de tris-benzylidène-acétone-dipalladium-(0) et 1 g de triphénylphosphine. Ensuite l'éther a été éliminé par distillation sous balayage d'argon sec, et le mélange maintenu à 70 °C pendant six heures. Le produit de la réaction a été filtré et la DMF a été éliminée à l'aide d'un évaporateur rotatif sous vide partiel à 60°C. Le résidu solide a été repris dans 100 ml d'eau et filtré. Le sel de tétraéthylammonium de la bis (trifluorovinylphenylsulfonimide) ) a été précipité par addition de 20 g de $(C_2H_5)_4NCl$ en solution dans 50 ml d'eau. Le produit brut a été recristallisé dans un mélange éthanol-eau. Il répond à la formule :

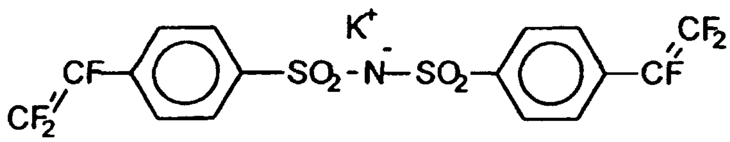

**Exemple 7**

On a dissout 302 g de chlorure d'iodophénylsulfonyl $IC_6H_4SO_2Cl$ ("pipsyl chloride" commercial) dans 1 1 d'acétonitrile à 25°C et on a ajouté 105 g de trifluorométhanesulfonamide et 225 g de 1,4-diazabicyclc [2,2,2]-octane (DABCO). Le mélange a été agité pendant 8 heures au cours desquelles un précipité de chlorhydrate de DABCO s'est formé. Le mélange réactionnel a ensuite été filtré, et le solvant évaporé. Le résidu solide a été repris dans 300 ml d'une solution saturée de chlorure de potassium et 100 ml d'acide acétique. On a séparé par filtration et recristallisé dans l'eau le précipité formé qui répond à la formule :

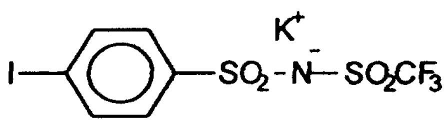

On a mis en solution dans 700 ml de DMF, 362,6 g du sel ainsi préparé, et on a ajouté 185 g du dérivé zincique dans l'éther préparé par un procédé similaire à celui décrit dans l'exemple 8, ainsi que 2,5 g de trisbenzilidène dipalladium (0) et 4 g de triphénylphosphine. L'éther a ensuite été distillé sous flux d'argon et le mélange a été maintenu à 60°C sous agitation pendant 5 heures. Le produit de la réaction a été filtré et la DMF éliminée à l'aide d'un évaporateur rotatif sous vide partiel à 60°C. Le résidu solide a été lavé à l'eau saturée de KCl, séché puis lavé au dichlorométhane. Le sel obtenu a été purifié par cristallisation dans un mélange eau-éthanol. Il répond à la formule :

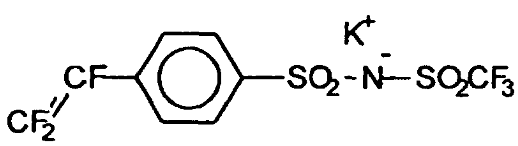

**Exemple 8**

40 g du monomère ionique monofonctionnel préparé selon le mode opératoire décrit dans l'exemple 7 et 2,9

g du monomère ionique difonctionnel préparé selon le mode opératoire de l'exemple 6 ont été dissous dans 100 ml de DMF. A la solution ainsi obtenue, on a ajouté 7,5 g de silice colloïdale [constituée de particules ayant une taille moyenne de 0,007 microns (Aldrich 38,126-8)] et 600 mg de 1,2-diphényl-1-kéto-2,2- diméthoxyéthane

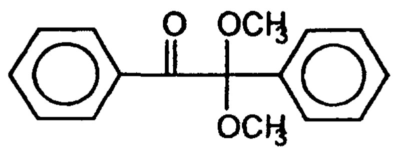

La suspension a été homogénéisée et dégazée par barbotage d'azote, puis épandue en une couche de 60 µm sur un film de polyéthylène téréphtalate (PET), et finalement soumise à une irradiation UV de 1 $W/cm^2$ produite par une lampe de type Hanovia, pendant 50 secondes. Le film a été maintenu sous couverture d'azote pendant l'exposition et le "post cure" de 5 minutes. La solution visqueuse s'est solidifiée pour former un film élastique. La DMF a été éliminée par étuvage pendant 48 heures à 80°C, ce qui a permis de séparer la membrane polyélectrolyte de son support de PET. La membrane a été lavée à l'eau acidulée (solution d'acide nitrique 1M) renouvelée plusieurs fois. Les ions (K + TEA) de la membrane ainsi obtenue ont été échangés par des protons. La membrane a été lavée à l'eau distillée puis séchée sous vide pour donner un film rigide ayant une épaisseur d'environ 18 µm et une très bonne tenue mécanique.

**Exemple 9**

On a synthétisé 100 g de $ClCF_2CFClSO_2F$ suivant la méthode décrite par Forohar Farhad ("Studies of the Chemistry of Perfluorovinylsulfonyl Fluoride", Clemson University, Thesis 1990 UMI 9115049).

Dans 50 ml de THF anhydre, on a mis en solution 10 mmoles de malononitrile fraîchement sublimé, puis on a porté la solution à 0°C. On a alors ajouté par portions, 20 mmoles d'hydrure de lithium LiH. Après 2 heures, on a ajouté 10 mmoles de $ClCF_2CFClSO_2F$. Après 48 heures, la solution a été centrifugée pour éliminer le précipité de LiF, puis l'on a ajouté 20 mmoles de zinc activé. Après 24 heures sous agitation, le THF a été évaporé et le résidu repris dans l'acétonitrile, puis filtré. Après évaporation de la solution filtrée et séchage, on a obtenu le composé suivant :

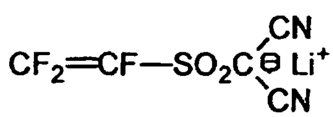

**EXEMPLE 10**

Dans 30 ml de THF anhydre à 0°C et sous argon contenant 20 mmoles de zinc, on a ajouté lentement 10 mmoles de l'iodure de trifluorovinyle $CF_2$=CFI. Après deux heures sous agitation, l'excès de zinc a été éliminé par filtration sous argon. On a alors ajouté à la solution de zincique 5 mmoles du sel de potassium de la bis(chlorosulfonyl) imide et 1 mmoles de $Pd[P(C_6H_5)_3]_4$ comme catalyseur. Après 24 heures sous agitation, le solvant a été évaporé puis le résidu recristallisé dans une solution saturée de chlorure de potassium. Après filtration et séchage, on a récupéré le composé suivant :

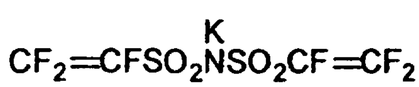

On a obtenu le sel de lithium par échange ionique avec du chlorure de lithium dans le tétrahydrofurane.

## Revendications

**1.** Membrane constituée par un copolymère d'au moins deux monomères choisis parmi les composés ioniques dans lesquels la charge négative est fortement délocalisée, **caractérisée en ce que** :

- l'un au moins des monomères est choisi parmi les monomères monofonctionnels suivants : $\{[CF_2{=}CF{-}SO_2NCN]^-\}_m\ M^{m+}$, $\{[CF_2{=}CF{-}SO_2C(CN)_2]^-\}_m\ M^{m+}$, $\{[CF_2{=}CFCF_2{-}SO_2NCN]^-\}_m\ M^{m+}$, $\{[CF_2{=}CFCF_2{-}SO_2C(CN)_2]^-\}_m\ M^{m+}$, $\{[CF_2{=}CF{-}\Phi SO_2NCN]^-\}_m\ M^{m+}$, $\{[CF_2{=}CF{-}\Phi SO_2C(CN)_2]^-\}_m\ M^{m+}$, $\{[CF_2{=}CF{-}\Phi SO_2NSO_2CF_3]^-\}_m\ M^{m+}$, $\{[CF_2{=}CF{-}\Phi SO_2C(SO_2CF_3)_2]^-\}_m\ M^{m+}$, $\{[CF_2{=}CF{-}\Phi SO_2CH(SO_2CF_3)]^-\}_m\ M^{m+}$,

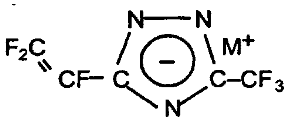
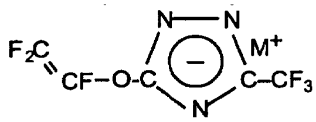
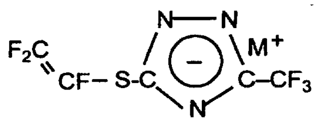
;

- l'un au moins des monomères est choisi parmi les monomères di- ou trifonctionnels suivants : $\{[(CF_2{=}CF{-}SO_2)_2N]^-\}_m\ M^{m+}$, $\{[(CF_2{=}CFCF_2{-}SO_2)_2N]^-\}_m\ M^{m+}$, $\{[(CF_2{=}CF{-}SO_2)_2CH]^-\}_m\ M^{m+}$, $\{[(CF_2{=}CFCF_2{-}SO_2)_2CH]^-\}_m\ M^{m+}$, $\{[(CF_2{=}CF{-}SO_2)_3C]^-\}_m\ M^{m+}$, $\{[(CF_2{=}CFCF_2{-}SO_2)_3C]^-\}_m\ M^{m+}$, $\{[(CF_2{=}CF{-}\Phi SO_2)_2N]^-\}_m\ M^{m+}$, $\{[(CF_2{=}CF{-}O{-}\Phi SO_2)_2N]^-\}_m\ M^{m+}$, $\{[(CF_2{=}CF{-}\Phi SO_2)_2CH]^-\}_m\ M^{m+}$, $\{[(CF_2{=}CF{-}O{-}\Phi SO_2)_2CH]^-\}_m\ M^{m+}$, $\{[(CF_2{=}CF{-}\Phi SO_2)_3C]^-\}_m\ M^{m+}$, $\{[(CF_2{-}CF{-}O{-}\Phi SO_2)_3C]^-\}_m\ M^{m+}$, $\{[(CF_2{=}CF{-})_2C_6H_3SO_3]^-\}_m\ M^{m+}$, $\{[3,5{-}(CF_2{=}CF{-})_2C_6H_3SO_2NSO_2CF_3]^-\}_m M^{m+}$

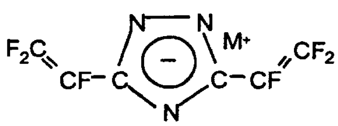
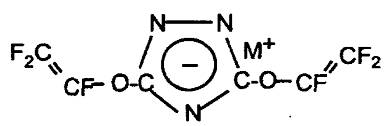
;

- $M^{m+}$ représentant, dans tous les monomères précités, le proton, ou un cation métallique ayant la valence m choisi parmi les ions des métaux alcalins, des métaux alcalino-terreux, des métaux de transition ou des terres rares, ou un cation organique onium, ou un cation organo-métallique, $1 \leq m \leq 3$ ;
- $\Phi$ représente un groupement aromatique condensé ou non, portant ou non un ou plusieurs substituants, contenant ou non des hétéroatomes, ou un groupement polyhalogéné $-C_6H_{(4-x-y)}F_xCl_y-$ ($1 \leq x+y \leq 4$).

**2.** Membrane selon la revendication 1, **caractérisée en ce que** $M^{m+}$ est un cation métallique choisi dans le groupe constitué par $Li^+$, $K^+$, $Mg^{++}$, $Ca^{++}$, $Ba^{++}$, $Cu^{++}$, $Zn^{++}$, $Fe^{++}$ et $Re^{+++}$.

**3.** Membrane selon la revendication 1, **caractérisée en ce que** $M^{m+}$ est un ammonium $[N(Y^j)_4]^+$, un amidinium $RC[N(Y^j)_2]_2^+$, un guanidinium $C[N(Y^j)_2]_3^+$, un pyridinium $[C_5N(Y^j)_6]^+$, un imidazolium $C_3N_2(Y^j)_5^+$, un imidazolinium $C_3N_2(Y^j)_7^+$, un triazolium $C_2N_3(Y^j)_4^+$, un carbonium $C_5(Y^j)_5C^+$, un $NO^+$ (nitrosyle), $NO_2^+$, un sulfonium $[S(Y^j)_3]^+$, un phosphonium $[P(Y^j)_4]^+$ ou un iodonium $[I(Y^j)_2]^+$, les substituants $Y^j$ d'un même cation, qui peuvent être identiques ou différents, représentant H ou un radical organique monovalent ayant de 1 à 16 atomes de carbone choisi parmi les radicaux alkyles, alkényles, oxa-alkyles, oxa-alkényles, aza-alkyles, aza-alkényles, aryles ou alkylaryles, ou parmi les radicaux obtenus à partir des radicaux précités par substitution, dans les chaînes et/ou la partie aromatique, par des hétéroatomes tels que les halogènes, l'oxygène, l'azote, le soufre, le phosphore, étant entendu que le soufre ou le phosphore sont présents, ils peuvent éventuellement être liés à des atomes d'oxygène ou d'azote substitués, ou bien $Y^j$ est une unité de répétition d'une trame polymérique.

**4.** Membrane selon la revendication 1, **caractérisée en ce que** $M^{m+}$ est un cation organo-métallique, choisi parmi les métallocéniums ; les cations métalliques coordonnés par des atomes tels que O, S, Se N, P ou As, portés par des molécules organiques, ces cations faisant éventuellement partie d'une trame polymérique ; ou un groupement trialkylsilyle, trialkylgermanyle ou trialkylstannyle.

**5.** Membrane selon la revendication 1, **caractérisée en ce que** le cation $M^+$ est l'unité de répétition d'un polymère conjugué sous forme oxydée cationique.

**6.** Membrane selon la revendication 1, **caractérisé en ce que** le radical divalent Φ est un phényle $C_6H_4$ correspondant aux positions ortho, méta et para de la substitution, ou un groupement aromatique, phényle substitué et / ou à noyaux condensés contenant ou non de hétéroatomes.

**7.** Utilisation d'une membrane selon l'une des revendications 1 à 6, dans un système de dialyse.

**8.** Utilisation d'une membrane selon l'une des revendications 1 à 6, comme électrolyte d'une pile à combustible.

**9.** Utilisation d'une membrane selon l'une des revendications 1 à 6 dans les procédés de traitement des effluents.

**Claims**

**1.** A membrane comprised of a copolymer of at least two monomers selected from ionic compounds in which the negative charge is highly delocalized, **characterized in that** :

- at least one of the monomers is selected from the following monofunctional monomers : $\{[CF_2{=}CF\text{-}SO_2NCN]^-\}_m\ M^{m+}$, $\{[CF_2{=}CF\text{-}SO_2C(CN)_2]^-\}_m\ M^{m+}$, $\{[CF_2{=}CFCF_2\text{-}SO_2NCN]^-\}_m\ M^{m+}$, $\{[CF_2{=}CFCF_2\text{-}SO_2C(CN)_2]^-\}_m\ M^{m+}$, $\{[CF_2{=}CF\text{-}\Phi SO_2NCN]^-\}_m\ M^{m+}$, $\{[CF_2{=}CF\text{-}\Phi SO_2C(CN)_2]^-\}_m\ M^{m+}$, $\{[CF_2{=}CF\text{-}\Phi SO_2NSO_2CF_3]^-\}_m\ M^{m+}$, $\{[CF_2{=}CF\text{-}\Phi SO_2C(SO_2CF_3)_2]^-\}_m\ M^{m+}$, $\{[CF_2{=}CF\text{-}\Phi SO_2CH(SO_2CF_3)]^-\}_m\ M^{m+}$ ;

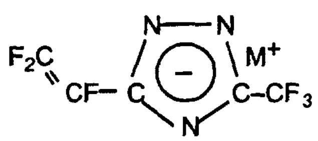
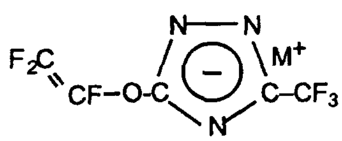
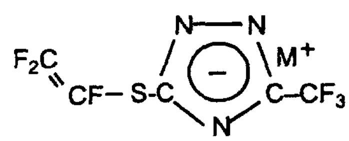

- at least one of the monomers is selected from the following di- or trifunctional monomers : $\{[(CF_2{=}CF\text{-}SO_2)_2N]^-\}_m\ M^{m+}$, $\{[(CF_2{=}CFCF_2\text{-}SO_2)_2N]^-\}_m\ M^{m+}$, $\{[(CF_2{=}CF\text{-}SO_2)_2CH]^-\}_m\ M^{m+}$, $\{[(CF_2{=}CFCF_2\text{-}SO_2)_2CH]^-\}_m\ M^{m+}$, $\{[(CF_2{=}CF\text{-}SO_2)_3C]^-\}_m\ M^{m+}$, $\{[(CF_2{=}CFCF_2\text{-}SO_2)_3C]^-\}_m\ M^{m+}$, $\{[(CF_2{=}CF\text{-}\Phi SO_2)_2N]^-\}_m\ M^{m+}$, $\{[(CF_2{=}CF\text{-}O\text{-}\Phi SO_2)_2N]^-\}_m\ M^{m+}$, $\{[(CF_2{=}CF\text{-}\Phi SO_2)_2CH]^-\}_m\ M^{m+}$, $\{[(CF_2{=}CF\text{-}O\text{-}\Phi SO_2)_2CH]^-\}_m\ M^{m+}$, $\{[(CF_2{=}CF\text{-}\Phi SO_2)_3C]^-\}_m\ M^{m+}$, $\{[(CF_2{=}CF\text{-}O\text{-}\Phi SO_2)_3C]^-\}_m\ M^{m+}$, $\{[(CF_2{=}CF\text{-})_2C_6H_3SO_3]^-\}_m\ M^{m+}$, $\{[3,5\text{-}(CF_2{=}CF\text{-})_2C_6H_3SO_2NSO_2CF_3]^-\}_m M^{m+}$

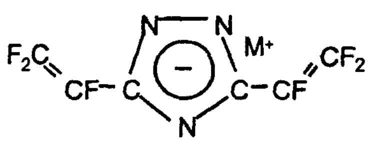
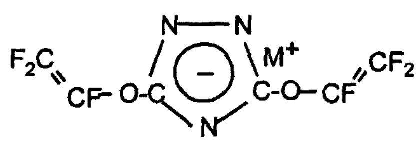
;

- $M^{m+}$ represents, in each monomer, a proton or a metal cation having the valency m chosen from the alkali metal, alkaline earth metal, transition metal or rare earth metal ions, or an organic onium cation, or an organometallic cation, $1 \le m \le 3$;

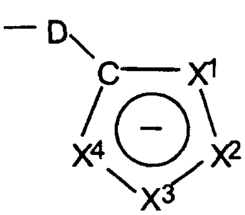
-

Φ represents a condensed or noncondensed aromatic group, which may or may not carry one or more substituents and which may or may not comprise heteroatoms, or a polyhalogenated group $-C_6H_{(4-x-y)}F_xCl_y-$ ($1 \le x+y \le 4$) .

**2.** A membrane according to Claim 1, **characterized in that** $M^{m+}$ is a metal cation chosen from the group consisting of $Li^+$, $K^+$, $Mg^{++}$, $Ca^{++}$, $Ba^{++}$, $Cu^{++}$, $Zn^{++}$, $Fe^{++}$ and $Re^{+++}$.

**3.** A membrane according to Claim 1, **characterized in that** $M^{m+}$ is an ammonium $[N(Y^j)_4]^+$, an amidinium $RC[N(Y^j)_2]_2^+$, a guanidinium $C[N(Y^j)_2]_3^+$, a pyridinium $[C_5N(Y^j)_6]^+$, an imidazolium $C_3N_2(Y^j)_5^+$, an imidazolinium $C_3N_2(Y^j)_7^+$, a triazolium $C_2N_3(Y^j)_4^+$, a carbonium $C_5(Y^j)_5C^+$, an $NO^+$ (nitrosyl), $NO_2^+$, a sulfonium $[S(Y^j)_3]^+$, a phosphonium $[P(Y^j)_4]^+$ or an iodonium $[I(Y^j)_2]^+$, the $Y^j$ substituents of the same cation, which can be identical or different, representing H or a monovalent organic radical having from 1 to 16 carbon atoms chosen from alkyl, alkenyl, oxaalkyl, oxaalkenyl, azaalkyl, azaalkenyl, aryl or alkylaryl radicals or from the radicals obtained from the abovementioned radicals by substitution, in the chains and/or the aromatic part, by heteroatoms, such as halogens, oxygen, nitrogen, sulfur or phosphorus, it being understood that, if sulfur or phosphorus are present, they can optionally be bonded to substituted nitrogen or oxygen atoms, or else $Y^j$ is a repeat unit of a polymeric backbone.

**4.** A membrane according to Claim 1, **characterized in that** $M^{m+}$ is an organometallic cation chosen from metalloceniums; metal cations coordinated by atoms, such as O, S, Se, N, P or As, carried by organic molecules, these cations optionally forming part of a polymeric backbone; or a trialkylsilyl, trialkylgermanyl or trialkylstannyl group.

**5.** A membrane according to Claim 1, **characterized in that** the $M^+$ cation is the repeat unit of a conjugated polymer in cationic oxidized form.

**6.** A membrane according to Claim 1, **characterized in that** the divalent radical Φ is a phenyl $C_6H_4$ corresponding to the ortho, meta and para positions of substitution or an aromatic group, a phenyl which is substituted and/or comprising condensed nuclei which may or may not comprise heteroatoms.

**7.** The use of a membrane according to any of claims 1 to 6, in a dialysis system.

**8.** The use of a membrane according to any of claims 1 to 6, as the electrolyte in a fuel cell.

**9.** The use of a membrane according to any of claims 1 to 6, in a process for the treatment of effluents.

## Patentansprüche

**1.** Membran, bestehend aus einem Copolymer aus zumindest zwei Monomeren, die aus ionischen Verbindungen mit stark delokalisierter negativer Ladung ausgewählt sind, **dadurch gekennzeichnet, dass**:

- zumindest eines der Monomere aus den folgenden monofunktionellen Monomeren ausgewählt ist: $\{[CF_2=CF-SO_2NCN]^-\}_m$ $M^{m+}$, $\{[CF_2=CF-SO_2C(CN)_2]^-\}_m$ $M^{m+}$, $\{[CF_2=CFCF_2-SO_2NCN]^-\}_m$ $M^{m+}$, $\{[CF_2=CFCF_2-SO_2C(CN)_2]^-\}_m$ $M^{m+}$, $\{[CF_2=CF-\Phi SO_2NCN]^-\}_m$ $M^{m+}$, $\{[CF_2=CF-\Phi SO_2C(CN)_2]^-\}_m$ $M^{m+}$, $\{[CF_2=CF-\Phi SO_2NSO_2CF_3]^-\}_m$ $M^{m+}$, $\{[CF_2=CF-\Phi SO_2C(SO_2CF_3)_2]^-\}_m$ $M^{m+}$, $\{[CF_2=CF-\Phi SO_2CH(SO_2CF_3)]^-\}_m$ $M^{m+}$,

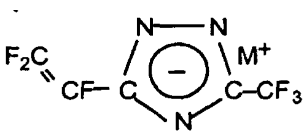 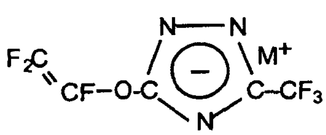 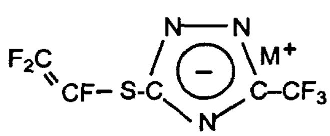 ;

- zumindest eines der Monomere aus den folgenden bioder trifunktionellen Monomeren ausgewählt ist: $\{[(CF_2=CF-SO_2)_2N]^-\}_m$ $M^{m+}$, $\{[(CF_2=CFCF_2-SO_2)_2N]^-\}_m$ $M^{m+}$, $\{[(CF_2=CF-SO_2)_2CH]^-\}_m$ $M^{m+}$, $\{[(CF_2=CFCF_2-SO_2)_2CH]^-\}_m$ $M^{m+}$, $\{[(CF_2=CF-SO_2)_3C]^-\}_m$ $M^{m+}$, $\{[(CF_2=CFCF_2-SO_2)_3C]^-\}_m$ $M^{m+}$, $\{[(CF_2=CF-\Phi SO_2)_2N]^-\}_m$ $M^{m+}$, $\{[(CF_2=CF-O-\Phi SO_2)_2N]^-\}_m$ $M^{m+}$, $\{[(CF_2=CF-\Phi SO_2)_2CH]^-\}_m$ $M^{m+}$, $\{[(CF_2=CF-O-\Phi SO_2)_2CH]^-\}_m$ $M^{m+}$, $\{[(CF_2=CF-\Phi SO_2)_3C]^-\}_m$ $M^{m+}$, $\{[(CF_2=CF-O-\Phi SO_2)_3C]^-\}_m$ $M^{m+}$, $\{[CF_2=CF-)_2C_6H_3SO_3]^-\}_m$ $M^{m+}$, $\{[3,5-(CF_2=CF-)_2C_6H_3SO_2NSO_2CF_3]^-\}_m M^{m+}$

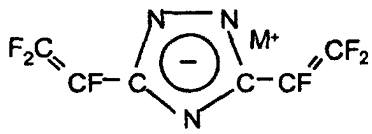 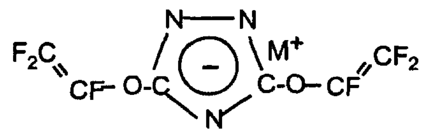

- $M^{m+}$ in allen der oben stehenden Monomere für ein Proton oder ein Metallkation mit der Wertigkeit m, ausgewählt aus Ionen von Alkalimetallen, Erdalkalimetallen, Übergangsmetallen und Seltenerdmetallen, oder für ein organisches Onium-Kation oder ein Organometallkation steht, $1 \leq m \leq 3$;
- Φ für eine aromatische, kondensierte oder nichtkondensierte Gruppierung, die keinen, einen oder B0361epde - 2119 mehrere Substituenten trägt und gegebenenfalls Heteroatome umfasst, oder eine polyhalogenierte Gruppierung $-C_6H_{(4-x-y)}F_xCl_y-$ $(1 \leq x+y \leq 4)$ steht.

**2.** Membran nach Abspruch 1, **dadurch gekennzeichnet, dass** $M^{m+}$ ein aus der aus $Li^+$, $K^+$, $Mg^{++}$, $Ca^{++}$, $Ba^{++}$, $Cu^{++}$, $Zn^{++}$, $Fe^{++}$ und $Re^{+++}$ bestehenden Gruppe ausgewähltes Metallkation ist.

**3.** Membran nach Abspruch 1, **dadurch gekennzeichnet, dass** $M^{m+}$ ein Ammonium $[N(Y^j)_4]^+$, ein Amidinium $RC[N(Y^j)_2]_2^+$, ein Guanidinium $C[N(Y^j)_2]_3^+$, ein Pyridinium $[C_5N(Y^j)_6]^+$, ein Imidazolium $C_3N_2(Y^j)_5^+$, ein Imidazolinium $C_3N_2(Y^j)_7^+$, ein Triazolium $C_2N_3(Y^j)_4^+$, ein Carbonium $C_5(Y^j)_5C^+$, ein $NO^+$ (Nitrosyl), $NO_2^+$, ein Sulfonium $[S(Y^j)_3]^+$, ein Phosphonium $[P(Y^j)_4]^+$ oder Iodonium $[I(Y^j)_2]^+$ ist, wobei die Substituenten $Y^j$ eines dieser Kationen, die identisch oder anders sein können, für H oder eninen einwertigen organischen Rest mit 1 bis 16 Kohlenstoffatomen stehen, der aus Alkyl-, Alkenyl-, Oxaalkyl-, Oxaalkenyl-, Aryl- oder Alkylarylresten oder aus jenen Resten ausgewählt ist, die ausgehend von den obigen Resten durch Substitution in den Ketten und/oder dem Aromatenanteil mit Heteroatomen, wie z.B. Halogenen, Sauerstoff, Stickstoff, Schwefel und Phosphor, erhalten werden, wobeil es sich versteht, dass, wenn Schwefel und Phosphor enthalten sind, diese gegebenenfalls an substituierte Sauerstoff- oder Stickstoffatome gebunden sind, oder worin $Y^j$ eine Grundeinheit eines Polymerferüstes ist.

**4.** Membran nach Anspruch 1, **dadurch gekennzeichnet, dass** $M^{m+}$ ein Organometall-Kation ist, das aus Metallocenium-Kationen; Kationen, die mit Atomen wie etwa 0, S, Se, N, P oder As koordiniert sind und von organischen Molekülen getragen werden, wobei diese Kationen gegebenenfalls Teil eines Polymergerüsts sind; oder Trialkylsilyl-, Trialkylgermanyl- und Trialkylstannylgruppierungen ausgewählt ist.

**5.** Membran nach Anspruch 1, **dadurch gekennzeichnet, dass** das Kation $M^+$ eine Grundeinheit eines konjugierten Polymers in kationisch oxidierter Form ist.

**6.** Membran nach Anspruch 1, **dadurch gekennzeichnet, dass** der zweiwertige Rest Φ ein entsprechend ortho-, metaoder para-substituiertes Phenyl $C_6H_4$ oder eine aromatische Gruppierung ist, die ein substituiertes Phenyl ist und/oder kondensierte Kerne aufweist und gegebenenfalls Heteroatome enthält.

**7.** Verwendung einer Membran nach einem der Ansprüche 1 bis 6 in einem Dialysesystem.

**8.** Verwendung einer Membran nach einem der Ansprüche 1 bis 6 als Elektrolyt einer Brennstoffzelle.

**9.** Verwendung einer Membran nach einem der Ansprüche 1 bis 6 in Verfahren zur Abwasserbehandlung.